Europäisches Patentamt

European Patent Office

Office européen des brevets

(19)

(11) Numéro de publication: **0 178 234**
A1

## (12) DEMANDE DE BREVET EUROPEEN

(21) Numéro de dépôt: 85420156.3

(22) Date de dépôt: 30.08.85

(51) Int. Cl.⁴: **A 61 K 6/02,** A 61 C 8/00,
A 61 C 13/30, A 61 F 2/30,
A 61 M 1/00

(30) Priorité: 31.08.84 FR 8413947

(43) Date de publication de la demande: 16.04.86
Bulletin 86/16

(84) Etats contractants désignés: AT BE CH DE FR GB IT LI
LU NL SE

(71) Demandeur: Russier, Jean-Jacques, 11 avenue du
Champ de Mars, F-26000 Valence (FR)

(72) Inventeur: Russier, Jean-Jacques, 11 avenue du Champ
de Mars, F-26000 Valence (FR)

(74) Mandataire: Maureau, Bernard et al, Cabinet GERMAIN
& MAUREAU Le Britannia - Tour C 20, Boulevard Eugène
Déruelle, F-69003 Lyon (FR)

(54) Joint physiologique étanche pour implants endo-extracorporels.

(57) Ce joint (3, 3a) est réalisé par un matériau d'attache
d'origine dentaire fibro et/ou ostéo-inducteur prévu pour
susciter et/ou permettre une fixation des tissus conjonctifs
et/ou osseux voisins sur l'implant, ce matériau étant choisi
parmi la dentine ou sa matrice organique, l'émail ou sa matrice organique.

## JOINT PHYSIOLOGIQUE ETANCHE
## POUR IMPLANTS ENDO-EXTRACORPORELS

La présente invention concerne un joint physiologique étanche assurant l'attachement mécanique d'implants endo-extracorporels aux tissus proximaux.

On connaît les nombreux problèmes rencontrés quand il s'agit d'introduire un corps étranger ou implant dans l'organisme.

De gros problèmes peuvent notamment apparaître lorsque ce corps étranger est en communication avec le milieu extérieur.

En effet, la ou les zones où cet implant traverse les tissus de recouvrement sont autant de portes d'entrée bactériennes, et tout se passe bien souvent comme si cette agression microbienne s'accompagnait d'une invagination épithéliale progressant le long de l'implant et visant à peu à peu à isoler puis exclure celui-ci de l'organisme.

La présente invention s'est donné pour but de pallier ces inconvénients en proposant un joint physiologique étanche aux points d'effraction muqueux, gingivaux ou cutanés de l'implant, joint qui supprime le problème capital de la porte d'entrée bactérienne cité ci-dessus et qui, par sa présence, induit, notamment en ce point ou à son voisinage immédiat, un attachement mécanique fonctionnel des tissus proximaux principalement des tissus mous sur les implants.

Différents types de joints ou de barrières bactériennes ont déjà été proposés.

C'est ainsi que le brevet US 3 663 965 décrit un dispositif percutané destiné à faciliter le passage de fils ou tubes au travers de la peau de telle sorte que soit réalisée une barrière étanche aux bactéries, la matière constituant la barrière étant choisie parmi le téflon, le caoutchouc de silicone, le polypropylène, le polyuréthane, les époxys, et diverses formes de carbone pyrolisé.

L'invagination épithéliale se fait en totalité autour de ce dispositif.

La rétention de l'ensemble n'est assurée que grâce à la colonisation épithéliale d'un ensemble sophistiqué de perforations macroscopiques du support. Ces perforations ont pour rôle, en outre, de ralentir la progression de la plaque bactérienne dans l'espace compris entre l'épithélium et le corps étranger, espace dont le nettoyage ne semble pas possible.

Le EP-A- 0 039 189 décrit un appareil hypodermique comprenant un bouton dont la jupe supérieure aussi bien que la jupe inférieure est constituée de polytétrafluoréthylène expansé présentant une microstructure telle qu'elle permette une pénétration tissulaire tridimensionnelle. On note, là aussi, la nature inerte du matériau constituant le bouton.

Le joint. physiologique étanche selon l'invention est réalisé en un matériau d'attache d'origine dentaire fibro et/ou ostéo-inducteur prévu pour susciter et/ou permettre une fixation des tissus conjonctifs et/ou osseux voisins sur l'implant.

La liaison se fait par un attachement fibreux éventuellement calcifié du tissu conjonctif voisin, les fibres se formant en continuité avec celles du joint. Cette organisation est compétente pour stopper l'invagination épithéliale autour de l'implant et éviter la formation d'une poche bactérienne, ce qui permet une maintenance aisée et efficace de la zone d'effraction muqueuse ou cutanée ; la continuité fibreuse semble être le moyen de rétention le plus efficace dont dispose l'organisme au niveau des tissus mous.

Selon un mode de réalisation de l'invention, le matériau d'attache est la dentine, ou sa matrice organique.

Dans le cas d'implant endo-osseux, le joint peut être double, à la fois fibro et/ou ostéoinducteur en relation avec le tissu conjonctif fibreux et/ou le périoste ; il est important que soit évité le contact direct entre la dentine et l'os et il convient donc d'intercaler entre ces deux éléments une couche de matière barrière convenable qui peut être choisie parmi les métaux ou alliages, les biocéramiques, le carbone vitrifié, les polymères...

La partie du joint ostéoinductrice en relation avec l'os peut être de l'émail dentaire ou sa matrice organique.

Dans le cas d'implant juxtaosseux sous périosté, la liaison peut être étendue entre la matrice organique de la dentine et le périoste, voire, s'il est absent, la fibro muqueuse.

La présente invention sera mieux comprise d'ailleurs et ses avantages ressortiront bien de la description qui suit, qui décrit, à titre d'exemples non limitatifs, deux modes de réalisation selon l'invention en référence au dessin schématique annexé, dans lequel :

- Figure 1 est une coupe transversale d'un implant dentaire juxtaosseux avec un seul poste (pilier d'ancrage prothétique) revêtu du joint physiologique selon l'invention ;

- Figure 2 est une vue similaire à figure 1 mais représentant un implant endo-osseux.

- Figure 3 est une vue en coupe similaire à figure 2 d'un mode d'exécution permettant un réglage de la hauteur du joint ou un renouvellement éventuel de celui-ci sans dépose de l'implant ;

- Figure 4 est une vue en perspective avec coupe partielle montrant l'application du joint selon l'invention sur un implant transcutané.

Sur les figures (2) désigne, de façon générale, l'implant et (3) le joint physiologique selon l'invention.

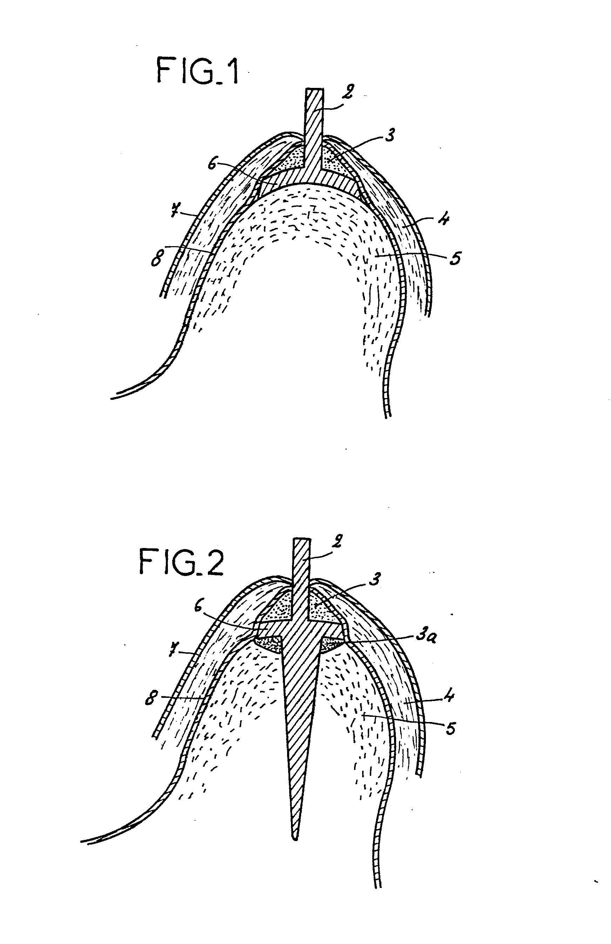

Sur les modes de réalisation représentés aux figures 1 à 3, la gencive est désignée par (4), l'os par (5), la collerette-support du joint par (6), l'épithélium gingival par (7) et le périoste par (8).

Le joint physiologique (3) selon l'invention est constitué de dentine obtenue à partir de dents non exposées à la plaque dentaire et conservées dans du sérum physiologique à une température voisine de - 20°C.

Cette dentine, partiellement décalcifiée par traitement superficiel de 3 à 5 minutes à l'acide citrique ou à l'acide ascorbique est découpée en un petit dôme de 4 à 5 mm de diamètre, évidé en son centre au diamètre standardisé du poste (2).

Un traitement prolongé à l'aide de ces mêmes acides permet d'obtenir l'ensemble de la matrice organique extracellulaire non calcifiée de la dentine.

Le processus est le même quand la dentine est remplacée par l'émail.

Afin d'éviter toute destruction de la dentine par les ostéoclastes, il convient que celle-ci ne soit pas au contact de l'os. Dans ce but, la dentine repose, par sa base, sur une collerette-support (6) qui la maintient à distance du contact avec l'os (5). Cette collerette (6) peut être réalisée dans la même matière que celle utilisée pour le poste (2) de l'implant ; elle peut être réalisée en toute matière barrière : métal, biocéramique, carbone vitrifié, polymères, etc...

Dans le cas d'un implant juxtaosseux, tel que celui représenté à la figure 1, le joint (3) est simple.

Dans le mode de réalisation représenté à la figure 2 (implant endo-osseux), le joint physiologique est double et comporte les parties (3) et (3a). La partie (3a), qui revêt la face de la collerette (6) en rapport direct avec l'os, est constituée par une mince couche d'émail préalablement traité comme la dentine dans le mode de réalisation précédent, c'est-à-dire décalcifié en surface ; cette partie (3a) complète ainsi la partie (3) en assurant un sertissage ostéo-inducteur.

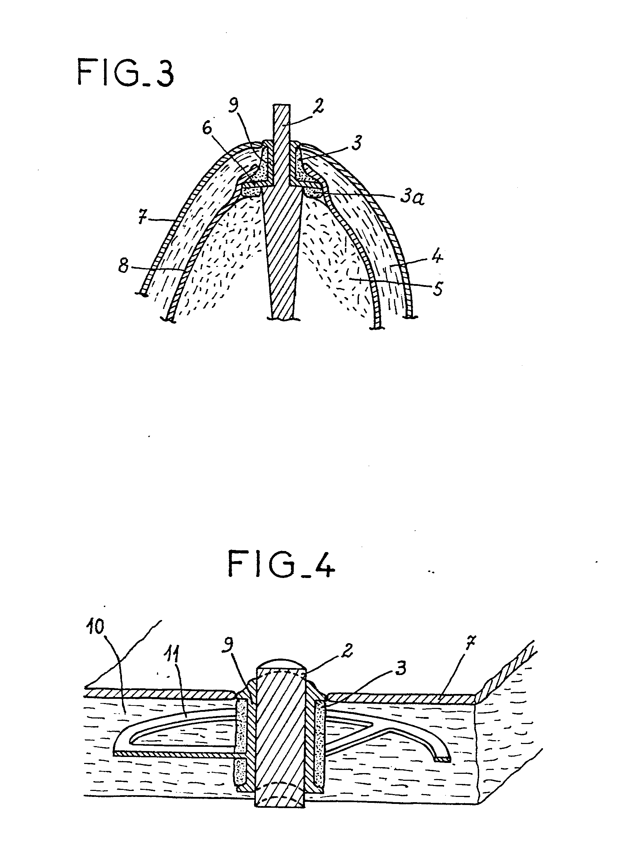

Dans le mode de réalisation représenté à la figure 3, le joint (3) n'est plus fixé directement sur l'implant (2) mais est fixé au préalable sur un manchon (9) coulissant sur l'implant et solidarisé secondairement par un produit assurant l'étanchéité. Ce manchon peut inclure, en outre, la collerette-support du joint (6). Ceci permet un réglage de la hauteur du joint en fonction des tissus voisins ainsi que son renouvellement éventuel sans déposer l'implant (2), ceci notamment en cas d'accident inflammatoire ou infectieux.

Le placement se fait de façon classique ; afin d'éviter tout risque d'invagination épithéliale, on contrôle, par tout moyen classique, l'épithélium (7) à proximité de la zone d'effraction, au début de la cicatrisation.

La dentine (3) ainsi que l'émail (3a) sont collés, de façon étanche, au support (6) par tout matériau classique neutre non cytotoxique.

On suture ensuite de façon à assurer un contact périosté intime avec la dentine.

On dispose ainsi d'un joint physiologique parfaitement étanche particulièrement au point d'effraction gingival et supprimant de ce fait tout problème d'entrée bactérienne. Il s'agit, en effet, d'une véritable attache de l'os et des tissus de recouvrement sur l'implant et, non pas d'un manchon épithélial évolutif drainant une infection chronique plus ou moins présente.

Le joint physiologique selon l'invention, constitué d'un tissu fibro et/ou ostéoinducteur, peut également, comme précisé ci-avant, être appliqué en implantologie en dehors du domaine dentaire puisqu'il assure une parfaite étanchéité au point d'effraction qu'il soit muqueux ou cutané.

C'est ainsi que dans le mode de réalisation représenté à la figure 4, le joint physiologique étanche (3) selon l'invention est plus spécialement décrit dans une application transcutanée. L'implant (2) peut être constitué par une pièce rigide, un fil, un tuyau souple ou tout autre dispositif per-ou transcutané autogène ou hétérogène.

Dans ce cas, la liaison se fait par un attachement fibreux entre le joint (3) et le tissu conjonctif (10) ; comme dans le mode de réalisation de la figure 3, le joint (3) n'est plus fixé directement sur l'implant (2) mais est fixé au préalable sur un manchon (9) coulisant sur l'implant et solidarisé secondairement par un produit assurant l'étanchéité.

Dans le cas d'un implant souple ou de tissus cutanés mobiles par rapport aux plans profonds, une ailette de stabilisation (11) prévue pour permettre de limiter le cisaillement des tissus est incorporée au dispositif.

Si cette ailette de stabilisation (11) est incluse dans le tissu conjonctif, elle doit présenter des orifices en taille et/ou en nombre suffisant pour permettre une bonne vascularisation de la couche la plus superficielle.

Cette ailette (11) est, de préférence, réalisée en un matériau dont la souplesse correspond essentiellement à celle du tissu de recouvrement.

Comme il va de soi et comme il ressort de ce qui précède, l'invention ne se limite pas aux seuls modes de réalisation décrits ci-dessus à titre d'exemples ; elle en embrasse, au contraire, toutes les variantes ; c'est ainsi que, dans le cas de postes plus importants, on peut utiliser ces matériaux durs sous forme agglomérée ou, au contraire, isoler de façon plus poussée le principe inducteur de la matrice extracellulaire de la dentine et de l'émail ; on peut également adjoindre au matériau d'origine dentaire une fibronectine ou un collagène, améliorant ainsi l'attachement fibreux.

6

## - REVENDICATIONS -

1- Joint (3,3a) pour implants endo-extracorporels, caractérisé en ce qu'il est réalisé par un matériau d'attache d'origine dentaire fibro et/ou ostéoinducteur prévu pour susciter et/ou permettre une fixation des tissus conjonctifs et/ou osseux voisins sur l'implant.

2- Joint selon la revendication 1, caractérisé en ce que le matériau d'attache est la dentine.

3- Joint selon la revendication 1 et la revendication 2, caractérisé en ce que le matériau d'attache est la matrice organique de dentine.

4- Joint selon la revendication 1, caractérisé en ce que le matériau d'attache est l'émail.

5- Joint selon la revendication 4, caractérisé en ce que le matériau d'attache est la matrice organique de l'émail.

6- Joint selon l'une quelconque des revendications 1 à 5, caractérisé en ce qu'une fibronectine est adjointe au matériau d'attache.

7- Joint selon l'une quelconque des revendications 1 à 5, caractérisé en ce qu'un collagène est adjoint au matériau d'attache.

8- Dispositif d'implantation endo-extracorporelle, caractérisé en ce que l'implant est fixé à l'aide du joint (3,3a) selon l'une quelconque des revendications 1 à 7.

9- Dispositif selon la revendication 8, caractérisé en ce que le joint (3) est séparé de l'os (5) ou du tissu conjonctif (9) par une partie-support (6).

10- Dispositif selon la revendication 9, caractérisé en ce que la partie-support (6) est réalisée en un matériau inerte monobloc.

11- Dispositif selon les revendications 8 à 10, caractérisé en que le joint (3) est fixé au préalable sur un manchon (9) coulissant sur l'implant et est solidarisé secondairement par un produit assurant l'étanchéité.

12- Dispositif selon les revendications 8 à 11, caractérisé en ce que dans le cas d'un implant souple ou de tissus cutanés mobiles par rapport aux plans profonds, une ailette de stabilisation (11), prévue pour permettre de limiter le cisaillement des tissus, est incorporée au dispositif.

13- Dispositif selon la revendication 12, caractérisé en ce que l'ailette de stabilisation (11) présente des orifices en taille et/ou en nombre suffisant pour permettre une bonne vascularisation de la couche la plus superficielle.

FIG.1

FIG.2

FIG_3

FIG_4

**Office européen des brevets**

**RAPPORT DE RECHERCHE EUROPEENNE**

0178234

Numéro de la demande

EP 85 42 0156

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | US-A-3 663 965 (H.L. LEE)<br>* Colonne 1, lignes 27-60; colonne 2, lignes 19-59; colonne 4, lignes 20-34; colonne 5, lignes 50-56; revendications * | 1,6-13 | A 61 K 6/02<br>A 61 C 8/00<br>A 61 C 13/30<br>A 61 F 2/30<br>A 61 M 1/00 |
| | --- | | |
| D,Y | EP-A-0 039 183 (W.L. GORE & ASSOCIATES)<br>* Page 3, alinéa 3; page 5, alinéa 3; page 7, alinéas 4,5; revendications; figures * | 1,6-12 | |
| | --- | | |
| Y | FR-A-1 499 912 (CH. RODRIGUEZ et al.)<br>* En entier * | 1,9 | |
| | --- | | |
| Y | CH-A- 643 732 (H. SCHEICHER)<br>* Revendications 1,11 * | 1,6-9 | DOMAINES TECHNIQUES RECHERCHES (Int. Cl.4) |
| | ----- | | A 61 K<br>A 61 C<br>A 61 F<br>A 61 M<br>A 61 L |

Le présent rapport de recherche a été établi pour toutes les revendications

| Lieu de la recherche<br>LA HAYE | Date d'achèvement de la recherche<br>10-12-1985 | Examinateur<br>BERTE M.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82